# EUROPEAN PATENT SPECIFICATION

(11) **EP 3 100 705 B1**
(45) Date of publication and mention of the grant of the patent: **06.11.2019**
(21) Application number: 16172908.2
(22) Date of filing: 03.06.2016
(51) Int. Cl.: A61G 12/00, A47B 31/04, B62B 3/02

(54) **TROLLEY**
WAGEN
CHARIOT

(30) Priority: 05.06.2015 NL 2014920
(43) Date of publication of application: 07.12.2016
(73) Proprietor: Mulder, Johanna Christina Everdina, 1017 DM Amsterdam (NL)
(72) Inventor: Mulder, Johanna Christina Everdina, 1017 DM Amsterdam (NL)
(74) Representative: van Essen, Peter Augustinus

(56) References cited:
- EP-A1- 1 795 087
- FR-A1- 2 906 521
- GB-A- 2 497 132
- US-A1- 2011 220 604

## Description

### Field of the invention

The invention relates to a trolley, in particular to a home care trolley.

### Background of the invention

In general, many types of carts or trolleys for medical or home care have been suggested. Many of these trolleys have a working top and drawers for supplies.

In order to reduce the footprint of these trolleys, for making them for instance more easy to store or to stow away when no in use, several types of foldable trolleys have been suggested.

US2002096845 describes according to its summary a transportable medical cart includes a frame assembly and a plurality of panels removably mounted on the frame assembly including a horizontal top panel, a pair of vertical side panels and a pair of extension panels. The side panels are movable from a retracted position wherein the side panels are disposed in parallel planes perpendicular to the top panel to a non-retracted position wherein the side panels are disposed at an acute angle to the planes. The extension panels are movable from a non-extended position wherein the extension panels are disposed vertically over the side panels in the retracted position to an extended position wherein the extension panels are horizontally aligned with the top panel. The side panels are releasably locked to the extension panels, respectively, with the side panels in the non-retracted position and the extension panels supported thereon, respectively, in the extended position. A plurality of drawer assemblies are mounted to the frame assembly, each including a slide rail and a drawer supported on the slide rail by gravity. The slide rails are movable relative to the frame assembly to permit movement of the drawers between fully inserted and fully withdrawn positions. The drawers are removable from and replaceable on the slide rails via lifting and lowering motions, respectively. The transportable medical cart is capable of being assembled and disassembled in a minimum number of simple steps.

WO0028934 according to its summary describes a medical treatment trolley that can be folded between a raised working position and a folded transport and storage position including at least two work-surfaces jointed-to-pivot, one work-surface of which is firmly attached to a frame, while the other work-surface is arranged to be foldable. The frame includes a supporting frame, at least two legs equipped with wheels and moveable relative to the frame, and a linkage system that supports and connects the supporting frame to the leg equipped with wheels that is jointed to pivot with the foldable work-surface.

FR2906521 according to its abstract describes a trolley that has a rolling parallelepiped frame carrying a tray wall constituted of vertical panels carried by the frame. The frame has fixed and opening parts articulated to each other around a vertical axis on a periphery of the frame to take a closed position in which the frame has parallelepiped shape, and an opening position. Two sides of the parallelepiped frame located on both sides of articulation are in extension with each other in the opening position. The panels are arranged on the frame so as to be articulated around the same vertical axis. An independent claim is also included for an assembly of two service trolleys.

US2011220604 according to its abstract describes a collapsible rack that includes symmetric support frames composed of vertical bars and horizontal bars and top and bottom frames connected between the support frames in a movable manner. The top and bottom frames are composed of connected movable left and right boards, which have front and rear faces that define recessed channels. Spaced fitting holes are defined in the channels. Straight bars form pegs. The top and bottom frames are of movability to allow the top and bottom frames to be collapsed to a stacked flat configuration when the rack is not in use, so as to facilitate storage and transportation. When the rack is to be used, the top and bottom frames are expanded and the straight bars are set to have the pegs fit in the fitting holes defined in the left and right boards to fix the top and bottom frames in the expanded position.

EP1795087 according to its abstract describes a mobile desk comprising a wheel-arrangement that is intended to rest on a floor or the like, referring further to a drawing. According to the abstract "It is significant of the desk according to the present invention that it comprises at least two elements, that at least one of the elements defines a horizontal top surface, that the desk may assume a position where the elements are expanded, and that the desk may assume a position where the elements are folded."

GB2497132 according to its abstract describes a pair of wheel-mounted manually movable trolleys A and B are capable of being wheeled about independently of each other. A rear trolley B is provided with a pivoted connecting latch and the trolleys are configured such that they can be placed together and rigidly joined by engaging the latch such that the two trolleys can be moved as a single rigidly-connected unit. The two trolleys may have U-shaped base frames and, the rear base frame being configured to locate within an open rear end of the front base frame and provide a lower abutment. A top frame of the rear trolley B may provide an upper abutment with the upright members and of the front trolley. The latch includes a pair of hook elements and which engage over a bar carried by upright members and. Vertical movement of the rear trolley may be prevented by lugs on the base frame of the front trolley.

Most of these devices are not suited for use as trolley that can store articles, has a relatively small storage footprint and/or is simple in production and use.

### Summary of the invention

The current invention provides a that is relatively simple and cheap to produce. Further or alternatively, the invention provides a trolley that has a reduced footprint, making is easier to store or stow away, in particular in home care situations.

Hence, it is an aspect of the invention to provide a foldable trolley according to claim 1.

The foldable trolley provides a working space suitable for medical or healthcare treatment, while allowing compact storage. Alternatively, the trolley may be applied in for instance hotels for carrying for instance supplies for room cleaning. The trolley may also hold tools or maintenance supplies.

The series of storage containers may be two storage containers, one coupled to one trolley end and one storage container coupled to the other container end. Usually, however, there will be three or more storage containers. One storage container can for instance slide on another storage container in order to divide the forces or provide additional stability.

In an embodiment, the container ends may be coupled via rails. Usually, such rails are substantially normal (i.e., substantially perpendicular), with respect to the container ends, to allow them to move away from one another. Thus, the rails can also be seen as functionally normal.

In an embodiment, the trolley ends are mutually coupled via at least one telescopic rail, for allowing said trolley ends to slide away from one another and back to one another between said folded and extended position.

In an embodiment, the first trolley end is coupled to one end of said telescopic rail, and the second trolley end is coupled to an opposite end of said telescopic rail. This provides a simple construction. In a construction, one telescopic rail may be sufficient. In order to improve stability, two or more telescopic rails may be used. For instance, one or two telescopic rails can be provided in combination with sliding support of the storage containers on one another.

In an embodiment, the trolley further comprises a washbasin in one of said working top ends, in particular in said second working top end.

In an embodiment, in said folded position one of the working top ends is above the other working top end, in particular said first working top end is above said second working top end. Thus, it is possible to almost double the available working top area when going from the folded position to the extended position

In an embodiment, said trolley comprises at least three telescopic rails, in particular one telescopic rail at a lower end of said trolley. Furthermore, two telescopic rails can be provided at an upper end of said trolley. The two upper telescopic rails can be mutually parallel, in particular in one plane.

In an embodiment, one telescopic rail near a lower (in use) end of said trolley. Two telescopic rail can be attached to a lower surface of one of said working top ends. In an embodiment, opposite ends of that telescopic rail may hold bars perpendicular to the rail. The bars may hold wheels. Thus, a simple, ridged construction frame is possible. Trolley ends may de fixed to the bars of in another way to the telescopic rail.

In an embodiment, the trolley end comprise respective trolley end parts, in an embodiment said trolley end parts each formed from polymer material, in an embodiment each trolley end part having two castors attached.

In an embodiment, the trolley end parts comprise shell parts. This provides a ridged construction at minimal weight. For home healthcare, for instance, less weight makes the trolley more manoeuvrable or handable in home situations.

In an embodiment, the trolley further comprises a series of storage containers one above the other between said first trolley end and said second trolley end below said working top.

In an embodiment, each time one storage container is attached to said first trolley end and one storage container is attached to said second trolley end. Thus, easy access is possible to each storage container in the extended position, while the contents is neatly stored away in the folded position. The storage containers in an embodiment thus slide in between one another. Thus, storage containers alternatingly fit between one another. A vertical distance between storage containers matches a height of an intermediate storage container. In particular the alternating storage containers provide easy access and closure.

In an embodiment, the storage containers comprise a container bottom and container walls enclosing a storage space, wherein a container wall is attaches to said first or second trolley end. In an embodiment, each time one of said first or second trolley end forms at least part of said container wall.

In an embodiment, each storage container comprises a support part for supporting a storage container or working top part above.

In an embodiment, each support part comprising a guiding part, in an embodiment comprising at least one wheel, for guiding a storage container or working top part above. In an embodiment, a wheel is attached at or near an end of a storage container that is opposite the end of a storage container that is coupled to a trolley end.

In an embodiment, the storage containers comprise a complementary guiding part. In an embodiment, a guiding rail forms a complementary guiding part for the at least one wheel. For instance, a bottom of a storage container may be provided with an elongated recess. This runs between ends of the storage containers. Alternatively, bearings or other sliding parts may be used.

In an embodiment, container ends opposite the storage container end linked to one trolley end are linked to corresponding container ends of higher or lower container ends for providing an abutment at said extended position. This provides an easy stop.

In an embodiment, the trolley is substantially rectangular with said trolley ends defining opposite end planes of said trolley, and said trolley ends slidably coupled to one another for sliding away in substantially normal direction of said end planes.

A telescopic rail comprises extendible elements and are for instance also referred to as slides or runners. Similar rails are often used for instance for drawers. Known are 2-section slides, three section slides, and so on. These may also be referred to as telescopic runners or telescopic slides. Usually, two or more profile parts run one in the other, for instance coaxially. These profile parts may comprise bearing parts or wheels that make these parts slide more easily. As such, these telescopic rails are known to a skilled person.

The invention further or alternatively pertains to a foldable trolley comprising a first trolley end and an opposite second trolley end, said trolley ends mutually coupled for providing said trolley with an extended position and a folded position, a series of storage containers one above the other between said trolley ends, with at least one storage container comprising a storage container end linked to one trolley end and at least one second storage container comprising a storage container end linked to the opposite trolley end. This allows the trolley ends to be pulled apart. Furthermore, in this way it may be possible to at the same time provide access to the contents of the storage containers.

In an embodiment, the trolley further comprises a working top comprising a first working top part having a first working top side attached to said first trolley end and a second working top part having a second working top side attached to said second trolley end.

In an embodiment, the storage containers comprise an upper abutment resting against a bottom of a higher neighbouring container. The abutment may provide coupling of the trolley ends and even guiding of the trolley ends.

The term "substantially" herein, like in "substantially consists", will be understood by and clear to a person skilled in the art. The term "substantially" may also include embodiments with "entirely", "completely", "all", etc. Hence, in embodiments the adjective substantially may also be removed. Where applicable, the term "substantially" may also relate to 90% or higher, such as 95% or higher, especially 99% or higher, even more especially 99.5% or higher, including 100%. The term "comprise" includes also embodiments wherein the term "comprises" means "consists of".

The term "functionally" will be understood by, and be clear to, a person skilled in the art. The term "substantially" as well as "functionally" may also include embodiments with "entirely", "completely", "all", etc. Hence, in embodiments the adjective functionally may also be removed. The term "functionally" is intended to cover deviations in a feature to which it refers which are such that in the functional use of the feature in combination with other features it relates to in the invention, the combination of features is able to operate.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The devices or apparatus herein are amongst others described during operation. As will be clear to the person skilled in the art, the invention is not limited to methods of operation or devices in operation.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "to comprise" and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably programmed computer. In the device or apparatus claims enumerating several means, several of these means may be embodied by one and the same item of hardware. The mere fact that certain measures are recited in mutually different dependent claims does not indicate that a combination of these measures cannot be used to advantage.

The invention further applies to an apparatus or device comprising one or more of the characterising features described in the description and/or shown in the attached drawings. The invention further pertains to a method or process comprising one or more of the characterising features described in the description and/or shown in the attached drawings.

The various aspects discussed in this patent can be combined in order to provide additional advantages. Furthermore, some of the features can form the basis for one or more divisional applications.

### Brief description of the drawings

Embodiments of a device will now be described, by way of example only, with reference to the accompanying schematic drawings in which corresponding reference symbols indicate corresponding parts, and in which:
Figure 1 schematically a perspective view of a trolley in unfolded or extended position;
Figure 2 schematically shows a perspective view of the trolley of figure 1 in folded position;
Figure 3 schematically shows a side view of figure 2;
Figure 4 schematically shows the trolley of figure 1 in exploded view;
Figure 5 schematically showing an alternative embodiment of a trolley in unfolded or extended state;
Figure 6 the trolley of figure 5 in folded state, and
Figure 7 a detail of the trolley of figure 5.

The drawings are not necessarily on scale.

### Description of preferred embodiments

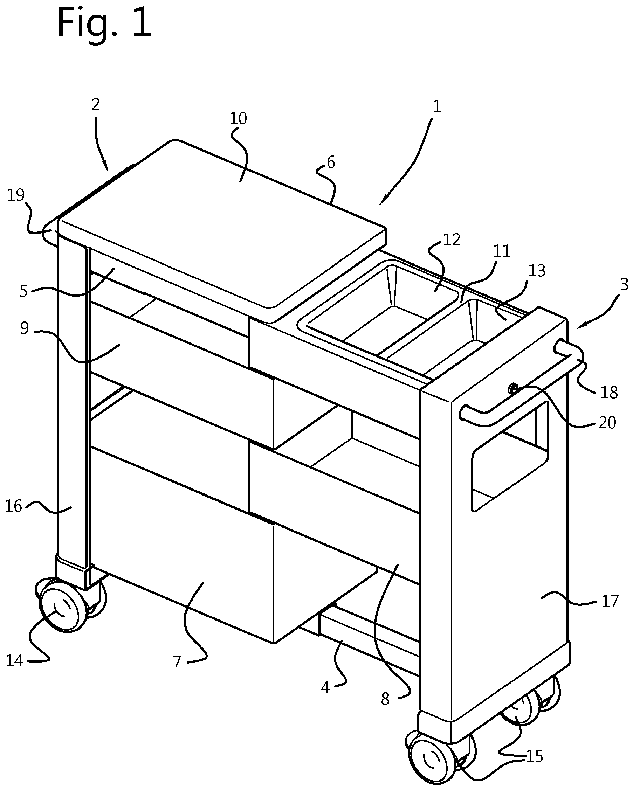

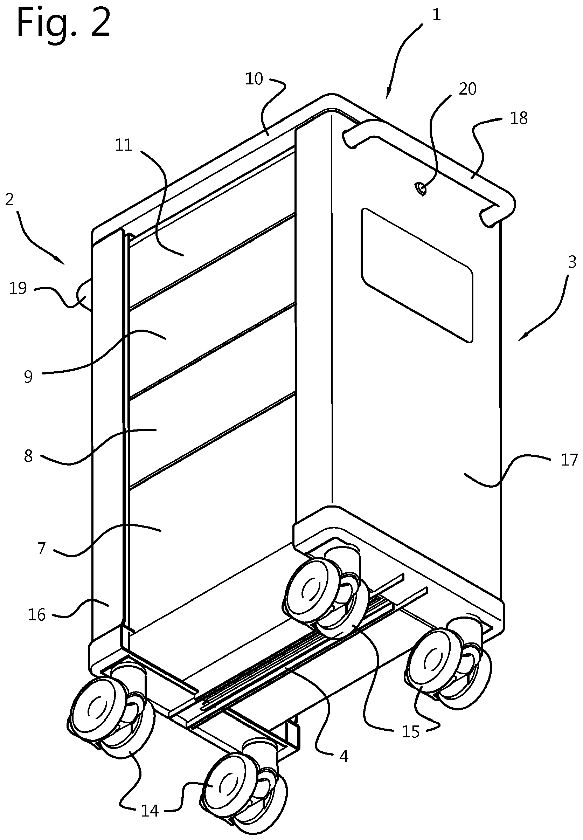

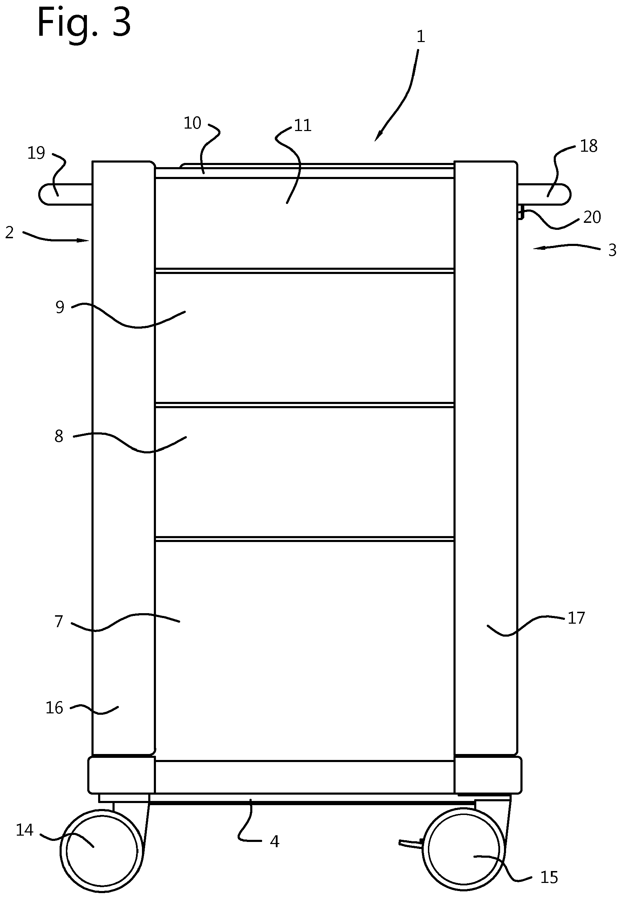

Figure 1 schematically depicts a trolley 1, in particular for home care. The trolley can be folded and unfolded. In figure 1 the trolley 1 is shown in its unfolded or extended position or state, and in figure 2 in its folded position or state, with figure 3 showing the trolley in folded state in a side view.

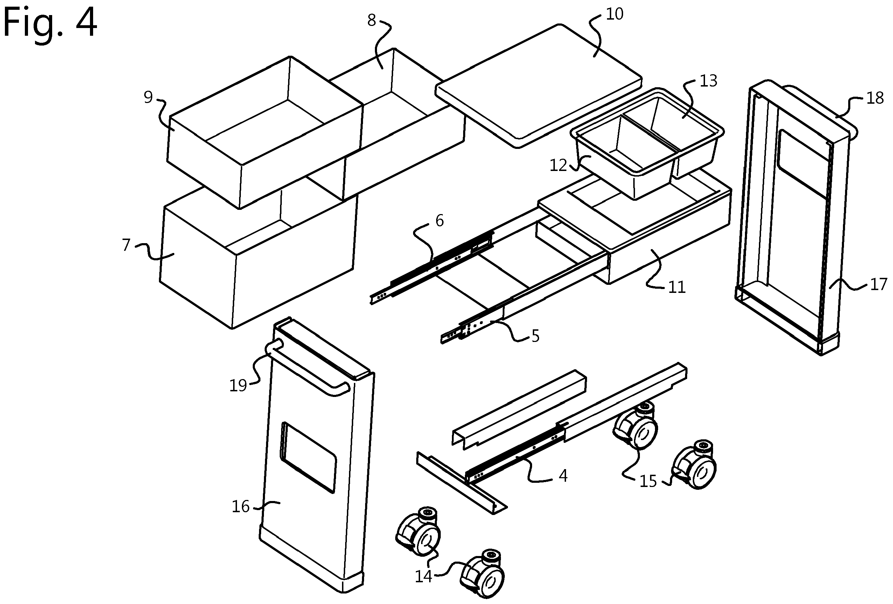

The trolley 1 has a first trolley end 2 and a second trolley end 3. The trolley ends 2, 3 are mutually coupled via telescopic rails 4, 5, 6. In this embodiment, three telescopic rails 4, 5, 6 are used. The telescopic rails as such are known to a skilled person. They should be strong enough for use in a trolley 1. The mutual positions and positions in the trolley 1 of the telescopic rails 4, 5, 6 is further illustrated in figure 4, showing the trolley in an exploded view. The telescopic rails 4, 5, 6 allow the trolley ends to move away from one another and back towards one another. Thus, a distance between the trolley ends 2, 3 can be varies between a folded distance, which can be between 50 cm and 1 meter, for instance, and an extended distance, which can be between 80 cm and 2 meters. A telescopic rail may comprise two or more profile ends with one running in the other. Thus, such a telescopic rail may extend between 1.5 and more of its shortest length. The telescopic rail may one or more wheels or it may comprise another mechanical bearing provision.

The trolley 1 further comprises a working top. The working top here comprises a first working top end 10 and a second working top end 11. In the extended of unfolded state, the working top ends are aligned and in fact functionally provide a single working top area. In this embodiment, the second working top end has a wash basin 12, 13, in particular two wash basins 12, 13. One of the wash basins 12, 13 may be used for clean water. Here, the first working top end provides a closed working surface. The first working top 10 in this embodiment is in the folded state above the second working top end 11. One end of the first working top end 10 is here linked to the first trolley end 2, and the second working top end is attached to the second working top end. When the trolley ends 2, 3 are moved from their unfolded state to their folded state, the first working top 10 here slides over the second working top end 11. In this embodiment, in order to provide additional strength, the telescopic rails 5, 6 may engage a lower surface of the first working top 10. Thus, when the trolley is unfolded or folded, the lower surface of the first working top 10 runs on an end of the telescopic rails 5, 6.

The second working top end here holds two wash basins 12, 13. The first working top end 10 in the folded state may cover, even close off, the wash basins 12, 13. This helps keeping them clean for use when the trolley 1 is stored. Furthermore, the height of the wash basins 12, 13 provides space for the telescopic rails 5, 6. In an embodiment, an end of the telescopic rail engages at least part of the second working top 11. That end of the telescopic rails 5, 6 may even be attached to the second working top end 11. Thus, additional strength may be provided, in particular as the part with the washing basins 12, 13 may be loaded, for instance when people rest on the second working top 11.

The trolley 1 further comprises a series of storage containers 7, 8, 9. There may be two or more storage containers, in practice it as found that there may be three to four storage containers. Here, three storage containers 7, 8, 9 are used. The storage containers are positioned one above the other in the folded state.

The storage containers have a storage container end. In this embodiment shown in the drawings, the storage containers 7, 8, 9 are rectangular. The storage containers 7, 8, 9 comprise a bottom and a peripheral wall.

One container end or peripheral wall part may be linked to a trolley end 2, 3. In an embodiment a wall part of a storage container may even be part or integrated in a trolley end2, 3. The container end may be linked, but also be attached, to the trolley end 2, 3. It may comprise links that can be detachable.

In an embodiment, storage containers 7, 8, 9 may slide over one another, for instance a outer bottom surface of a higher storage container may be provided with ends sliding over an upper end or edge or rim of its lower container. Thus, in the extended state the storage may even slide individually between trolley ends 2, 3.

Storage containers 7, 8, 9 may be removable from the trolley 1 in order to make it more accessible and make refiling or cleaning easier.

The storage containers 7, 8, 9 may be made from for instance metal of plastic material. In an embodiment, one of the walls is transparent or translucent.

With one end of each container attached to each time one selected from the first and second trolley ends, in the extended state the storage containers will be staggered as shown in figure 1. Thus, in the unfolded or extended state this provides access to interior of the storage containers 7, 8, 9. Additional access to storage containers 7, 8, 9 may be provided through openings in the trolley ends16, 17.

The trolley ends 2, 3 here comprise respective trolley end parts 16, 17. These container end parts 16, 17 may be produced in one piece, for instance a plate of wood, plastic or metal material. The container end parts 16, 17 may form shells, having an end wall and peripheral wall parts. This provides additional strength for the intended use. This can best be seen in figure 4. In an embodiment, the container end parts may be produced in a moulding process, for instance injection moulding, but other processes my be considered. For heavy use, alternatively the trolley end parts 16, 17 may be from for instance stainless steel, with ends that are bent inwardly providing the trolley end parts 16, 17 that are shown in figure 4.

The trolley ends 2, 3 at an end may be provided with wheels. In this embodiment, the trolley 1 is provided with castors 14, 15. In this embodiment, the lower ends of the trolley end parts 16, 17 are provided with wheels 14, 15. In this embodiment, wheels 14, 15 are provided on corner of the trolley ends. The wheels 14, 15 may be provided on a reinforced lower beam that is attached to the trolley end parts 16, 17. In the embodiment of figures 2 and 4, a lower telescopic rail 4 has its ends attached to respective lower beams of the trolley end parts 16, 17. These lower beams may be integrated into shell parts of the trolley end parts 16, 17.

Here, the trolley ends are provided with handles 18, 19.

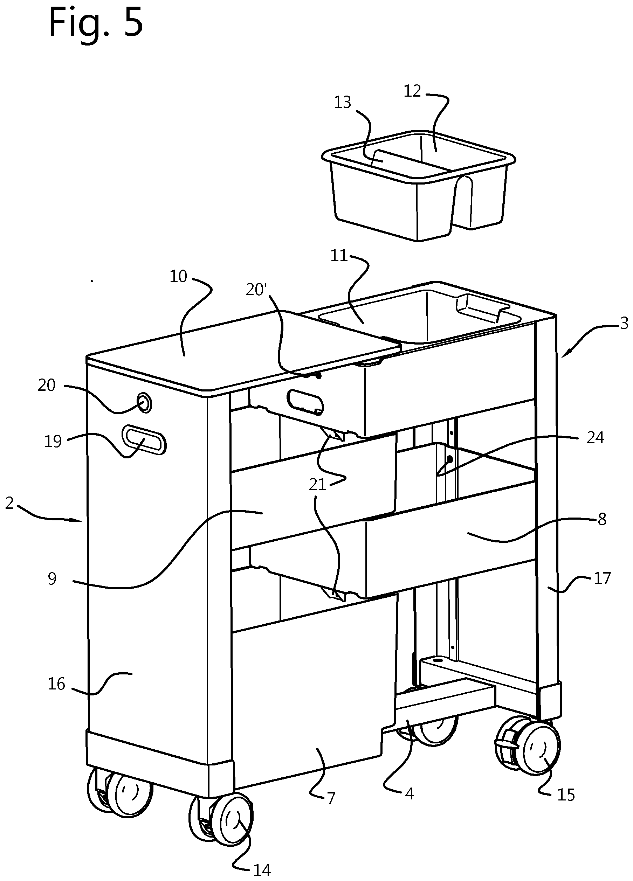

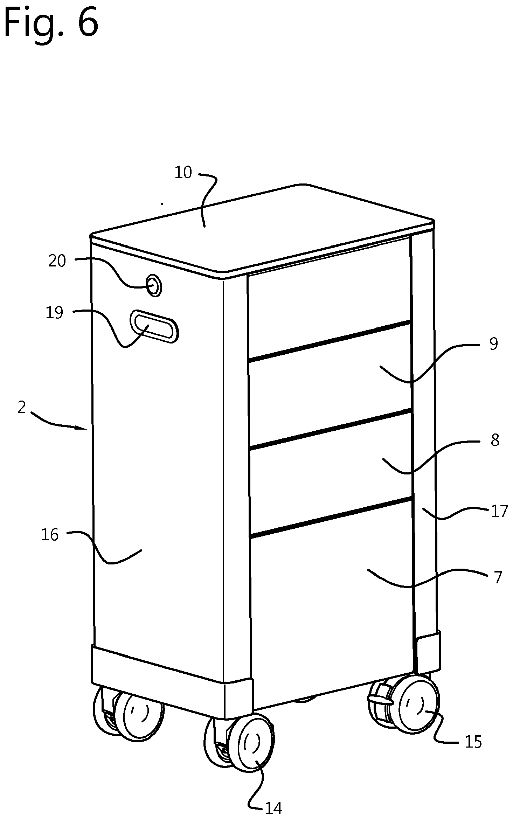

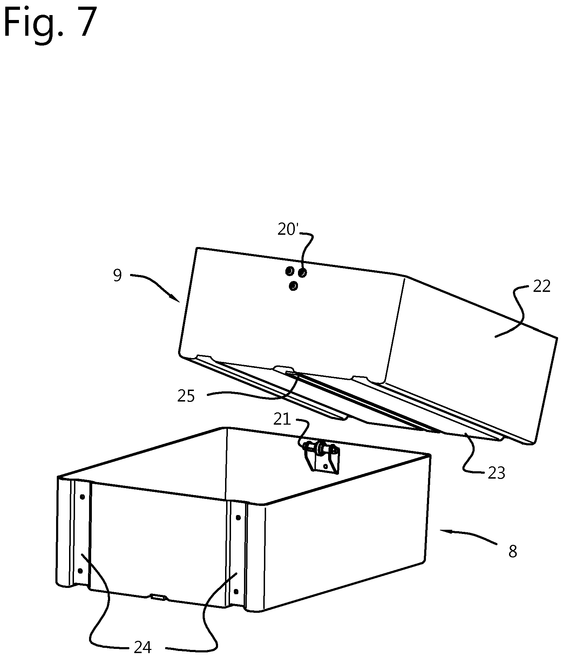

In figures 5-7, an alternative embodiment of the trolley is shown with figure 5 showing the trolley in its unfolded state or position, figure 6 showing the trolley in its folded position of state, and figure 7 showing a detail of that trolley, showing storage containers. In this embodiment, the trolley has only one telescopic rail, here only the lower telescopic rail 4. In this embodiment, the storage containers support one another. In this embodiment, each storage container is provided with a sliding support.

In the current embodiment, the storage containers 7-9 each comprise a wheel 21 as guiding part mounted at the end opposite of the peripheral wall 22 that is connected to a trolley end 2, 3. The storage containers comprise a lower storage container abutment. This provides an abutment for resting on a lower storage container. Here, a storage container is provided with a rail 25 for the wheel 21. The wheel 21 with the rail 25 in this embodiment provide a sliding support. In an alternative embodiment, a rim of a storage container may comprise an abutment and a bottom of a higher storage container may comprise a complementary abutment. In this way, the storage container rest on one another and are at one end fixed to a trolley end. The rim or the storage container bottom may be provided with a bearing means or the like, like a wheel or balls to form a bearing.

Here, the storage container end is provided with a coupling part that is indicated with reference number 24. Here, the trolley ends comprise strips and the storage container ends comprise indentations matching these strips. The storage containers are here fixed to the trolley ends. Alternatively, other coupling means may be used that may even allow removal of one or more storage containers. For instance, the storage containers may be hooked on the respective trolley end.

In the embodiments of the current drawings, the trolley is substantially rectangular. The trolley end parts 16, 17 provide substantially parallel end planes.

It will also be clear that the above description and drawings are included to illustrate some embodiments of the invention, and not to limit the scope of protection. Starting from this disclosure, many more embodiments will be evident to a skilled person. These embodiments are within the scope of protection and the essence of this invention and are obvious combinations of prior art techniques and the disclosure of this patent.

### Reference numbers

- 1: trolley
- 2: first trolley end
- 3: second trolley end
- 4: lower telescopic rail
- 5, 6: upper telescopic rails
- 7: lower storage container
- 8: middle storage container
- 9: upper storage container
- 10: first working top end
- 11: second working top end
- 12: wash basin
- 13: wash basin
- 14: first castor wheels
- 15: second castor wheels
- 16: first trolley end part
- 17: second trolley end part
- 18: second handle
- 19: first handle
- 20, 20': locking part
- 21: guiding part/
- 22: peripheral wall
- 23: storage container bottom
- 24: storage container end with coupling part
- 25: lower storage container abutment

## Claims

1. A foldable trolley (1), in particular a home care trolley, comprising:
- a first trolley end (2) and an opposite second trolley end (3), said trolley ends mutually coupled for providing said trolley with an extended position and a folded position, with said first and second trolley ends (2, 3) providing opposite trolley ends in said folded position and in said extended position;
- a series of storage containers (7, 8) one above the other, with each time one storage container comprising a storage container end linked to one trolley end and a next storage container comprising a storage container end linked to the opposite container end;
- a working top comprising a first working top part (10) having a first working top side attached to said first trolley end (2) and a second working top part (11) having a second working top side attached to said second trolley end (3).

2. The trolley (1) of claim 1, wherein said first and second trolley ends (2, 3) are coupled for allowing said trolley ends (2, 3) displace with respect to one another between said folded and extended position in directions along an imaginary connecting line.

3. The trolley (1) of claim 1 or 2, wherein said trolley ends (2, 3) are mutually coupled via at least one telescopic rail (4, 5, 6), for allowing said trolley ends to move away from one another and back to one another between said folded and extended position.

4. The trolley (1) of claim 3, wherein said first trolley end is coupled to one end of said telescopic rail, and the second trolley end is coupled to an opposite end of said telescopic rail.

5. The trolley (1) of any one of the preceding claims, further comprising a washbasin (12, 13) in one of said working top ends, in particular in said second working top end (11).

6. The trolley (1) of any one of the preceding claims, wherein in said folded position one of the working top ends is above the other working top end, in particular said first working top end (10 is above said second working top end (11).

7. The trolley (1) of any one of the preceding claims, wherein said trolley comprises at least three telescopic rails, in particular one telescopic rail at a lower end of said trolley and two telescopic rails at an upper end of said trolley.

8. The trolley (1) of any one of the preceding claims, wherein one telescopic rail near a lower (in use) end of said trolley, and two upper telescopic rails attached near an upper (in use) end of said trolley, in particular said two upper telescopic rails substantially parallel with respect to one another, more in particular said two upper telescopic rails attached to a lower surface of one of said working top ends.

9. The trolley (1) of any one of the preceding claims, wherein said trolley end comprise respective trolley end parts, in an embodiment said trolley end parts each formed from polymer material, in an embodiment each trolley end part having two castors attached, in particular wherein the trolley end parts comprise shell parts.

10. The trolley (1) of any one of the preceding claims, further comprising a series of said storage containers (7, 8, 9) one above the other between said first trolley end and said second trolley end below said working top, in particular wherein each time one storage container (7, 9) is attached to said first trolley end (2) and one storage container (8) is attached to said second trolley end (3).

11. The trolley (1) of any one of the preceding claims, wherein said storage containers comprise a container bottom (23) and container walls (22) enclosing a storage space, wherein a container wall is attaches to said first or second trolley end, in particular each time one of said first or second trolley end forms at least part of said container wall.

12. The trolley (1) of any one of the preceding claims, wherein each storage container comprises a support part for supporting a storage container or working top part above.

13. The trolley (1) of any one of the preceding claims, wherein each support part comprising a guiding part, in particular comprising at least one wheel, for guiding a storage container or working top part above, more in particular wherein the storage containers comprise a complementary guiding part, in particular a guiding rail for the at least one wheel.

14. The trolley (1) of any one of the preceding claims, wherein container ends opposite the storage container end linked to one trolley end are linked to corresponding container ends of higher or lower container ends for providing an abutment at said extended position.

15. The trolley (1) of any one of the preceding claims, wherein said trolley is substantially rectangular with said trolley ends defining opposite end planes of said trolley, and said trolley ends slidably coupled to one another for sliding away in substantially normal direction of said end planes.

## Patentansprüche

1. Ein faltbarer Wagen (1), insbesondere ein heimpflegewagen, der umfasst:
- ein erstes Wagenende (2) und ein gegenüberliegendes zweites Wagenende (3), wobei die Wagenenden miteinander gekoppelt sind um den Wagen mit einer ausgefalteten Position und einem gefalteten Position zu versehen, wobei das erste und das zweite Wagenende (2, 3) gegenüberliegenden Wagenenden versehen im gefalteten Position und im ausgefalteten Position;
- Lagerbehälter (7, 8) übereinander, wobei jeweils ein Lagerbehälter ein Lagerbehälterende umfasst, das mit einem Wagenende verbunden ist, und einen nächsten Lagerbehälter, der ein Lagerbehälterende umfasst, das mit dem gegenüberliegenden Behälterende verbunden ist;
- eine Arbeitsplatte mit einem ersten Arbeitsoberteil (10) mit einer an dem ersten Wagenende (2) angebrachten ersten Arbeitsoberseite und einem zweiten Arbeitsoberteil (11) mit einer zweiten Arbeitsoberseite, die an dem zweiten Wagenende befestigt ist (3).

2. Wagen (1) nach Anspruch 1, wobei das erste und das zweite Wagenende (2, 3) miteinander gekoppelt sind, um zu ermöglichen, dass sich die Wagenenden (2, 3) zwischen der gefalteten und der ausgefalteten Position in Richtungen entlang einer imaginären Verbindungslinie gegeneinander verschieben.

3. Wagen (1) nach Anspruch 1 oder 2, wobei die Wagenenden (2, 3) über mindestens eine Teleskopschiene (4, 5, 6) miteinander gekoppelt sind, um zu ermöglichen, dass sich die Wagenenden voneinander und zurück bewegen zwischen der gefalteten und der ausgefalteten Position.

4. Wagen (1) nach Anspruch 3, wobei das erste Wagenende mit einem Ende der Teleskopschiene verbunden ist und das zweite Wagenende mit einem gegenüberliegenden Ende der Teleskopschiene gekoppelt ist.

5. Wagen (1) nach einem der vorhergehenden Ansprüche, ferner umfassend ein Waschbecken (12, 13) in einem der oberen Arbeitsplatten, insbesondere in dem zweiten oberen Arbeitsplatte (11).

6. Wagen (1) nach einem der vorhergehenden Ansprüche, wobei sich in der gefalteten Position eines der oberen Arbeitsplatten über dem anderen oberen Arbeitsplatte befindet, insbesondere das erste obere Arbeitsplatte (10) über dem zweiten oberen Arbeitsplatte (11) liegt.

7. Wagen (1) nach einem der vorhergehenden Ansprüche, wobei der Wagen mindestens drei Teleskopschienen umfasst, insbesondere eine Teleskopschiene an einem unteren Ende des Wagens und zwei Teleskopschienen an einem oberen Ende des Wagens.

8. Wagen (1) nach einem der vorhergehenden Ansprüche, wobei eine Teleskopschiene in der Nähe eines unteren (im Gebrauch befindlichen) Endes des Wagens und zwei obere Teleskopschienen in der Nähe eines oberen (im Gebrauch befindlichen) Endes des Wagens angebracht sind, insbesondere zwei obere Teleskopschienen, die im Wesentlichen parallel zueinander sind, insbesondere sind die beiden oberen Teleskopschienen an einer unteren Oberfläche eines der oberen Arbeitsplatten befestigt.

9. Wagen (1) nach einem der vorhergehenden Ansprüche, wobei das Wagenende entsprechende Wagenendteile aufweist, wobei in einer Ausführungsform die Wagenendteile jeweils aus Polymermaterial gebildet sind, wobei in einem Ausführungsbeispiel jedes Wagenendteil zwei insbesondere angebrachte Rollen aufweist, insbesondere wobei die Wagenendteile Schalenteile umfassen.

10. Wagen (1) nach einem der vorhergehenden Ansprüche, ferner umfassend eine Reihe von den Lagerbehältern (7, 8, 9), die übereinander zwischen dem ersten Wagenende und dem zweiten Wagenende unter der Arbeitsplatte angeordnet sind, insbesondere jeweils wenn ein Lagerbehälter (7, 9) an dem ersten Wagenende (2) befestigt ist und ein Lagerbehälter (8) an dem zweiten Wagenende (3) angebracht ist.

11. Wagen (1) nach einem der vorhergehenden Ansprüche, wobei die Lagerbehälter einen Behälterboden (23) und Behälterwände (22) umfassen, die einen Lagerraum umschließen, wobei eine Behälterwand an dem ersten oder dem zweiten Wagenende befestigt ist, insbesondere bildet jedes Mal eines der ersten oder zweiten Wagenenden mindestens einen Teil der Behälterwand.

12. Wagen (1) nach einem der vorhergehenden Ansprüche, wobei jeder Lagerbehälter ein Auflageteil zum Tragen eines darüberliegenden Lagerbehälters oder Arbeitsplattes aufweist

13. Wagen (1) nach einem der vorangehenden Ansprüche, wobei jedes Trägerteil ein Führungsteil, dass insbesondere mindestens ein Rad umfasst, zum Führen eines Lagerbehälters oder eines Arbeitsplattes umfasst, insbesondere wobei die Lagerbehälter ein komplementäres Führungsteil umfassen, insbesondere eine Führungsschiene für das mindestens eine Rad.

14. Wagen (1) nach einem der vorangehenden Ansprüche, wobei Behälterenden, die dem Lagerbehälterende gegenüberliegen die mit einem Wagenende verbunden sind, mit entsprechenden Behälterenden von höheren oder unteren Behälterenden verbunden sind, um in der ausgefahrenen Position einen Anschlag zu schaffen.

15. Wagen (1) nach einem der vorhergehenden Ansprüche, wobei der Wagen im Wesentlichen rechteckig ist, wobei die Wagenenden gegenüberliegende Endebenen des Wagens definieren, und die Wagenenden verschiebbar miteinander gekoppelt sind, um in im Wesentlichen normaler Richtung des Endebenen wegzuschieben.

## Revendications

1. Chariot pliable (1), en particulier un chariot de soins à domicile, comprenant:
- une première extrémité de chariot (2) et une seconde extrémité de chariot opposée (3), lesdites extrémités de chariot se couplant de manière mutuelle pour fournir audit chariot une position étendue et une position plié, avec ses première extrémité et seconde extrémité fournissant des extrémités opposantes dans cette position étendue et cette position plié;
- une série de conteneurs de stockage (7, 8) superposés, avec à chaque fois un conteneur de stockage comprenant une extrémité de conteneur de stockage liée à une extrémité du chariot et un conteneur de stockage suivant comprenant une extrémité de conteneur de stockage liée à l'extrémité de conteneur opposée;
- une surface de travail comprenant une première partie de la surface de travail (10) ayant un premier côté de dessus de travail fixé à ladite première extrémité du chariot (2) et une deuxième partie de la surface de travail (11) ayant un deuxième côté du haut de travail attaché à ladite deuxième extrémité du chariot (3)

2. Chariot (1) selon la revendication 1, dans lequel lesdites première et seconde extrémités de chariot (2, 3) sont couplées pour permettre auxdites extrémités de chariot (2, 3) de se déplacer l'une par rapport à l'autre entre ladite position pliée et étendue dans des directions le long d'un imaginaire. ligne de connexion.

3. Chariot (1) selon la revendication 1 ou 2, dans lequel lesdites extrémités de chariot (2, 3) sont couplées mutuellement via au moins un rail télescopique (4, 5, 6) pour permettre auxdites extrémités de chariot de s'écarter l'une de l'autre et de revenir à l'arrière l'un à l'autre entre ladite position pliée et étendue.

4. Chariot (1) selon la revendication 3, dans lequel ladite première extrémité de chariot est couplée à une extrémité dudit rail télescopique et la seconde extrémité de chariot est couplée à une extrémité opposée dudit rail télescopique.

5. Chariot (1) selon l'une quelconque des revendications précédentes, comprenant en outre un lavabo (12, 13) dans l'une desdites extrémités de la surface de travail, en particulier dans ladite seconde extrémité surface de travail (11).

6. Chariot (1) selon l'une quelconque des revendications précédentes, dans lequel, dans ladite position pliée, l'une des extrémités de la surface de travail est au-dessus de l'autre extrémité de la surface de travail, en particulier ladite première extrémité de la surface de travail (10) est située au-dessus de ladite seconde extrémité de la surface de travail (11). ).

7. Chariot (1) selon l'une quelconque des revendications précédentes, dans lequel ledit chariot comprend au moins trois rails télescopiques, en particulier un rail télescopique à une extrémité inférieure dudit chariot et deux rails télescopiques à une extrémité supérieure dudit chariot.

8. Chariot (1) selon l'une quelconque des revendications précédentes, dans lequel un rail télescopique près d'une extrémité inférieure (en utilisation) dudit chariot et deux rails télescopiques supérieurs fixés à proximité d'une extrémité supérieure (en utilisation) dudit chariot, en particulier deux rails télescopiques supérieurs sensiblement parallèles l'un à l'autre, plus particulièrement lesdits deux rails télescopiques supérieurs fixés à une surface inférieure de l'une desdites surface de travail.

9. Chariot (1) selon l'une quelconque des revendications précédentes, dans lequel ladite extrémité du chariot comprend des parties d'extrémité de chariot respectives, dans un mode de réalisation, lesdites parties d'extrémité de chariot étant chacune constituées d'un matériau polymère, dans un mode de réalisation, chaque partie d'extrémité de chariot ayant deux roulettes attachées, en particulier dans lequel les parties d'extrémité du chariot comprennent des parties de coque.

10. Chariot (1) selon l'une quelconque des revendications précédentes, comprenant en outre une série de lesdites conteneurs de stockage (7, 8, 9) superposés entre ladite première extrémité du chariot et ladite seconde extrémité du chariot sous ladite surface de travail, en particulier dans lesquels chaque fois un conteneur de stockage (7, 9) est fixé à ladite première extrémité du chariot (2) et un conteneur de stockage (8) est fixé à ladite seconde extrémité du chariot (3).

11. Chariot (1) selon l'une quelconque des revendications précédentes, dans lequel lesdits conteneurs de stockage comprennent un fond de conteneur (23) et des parois de conteneur (22) renfermant un espace de stockage, dans lequel une paroi de conteneur est fixée à ladite première ou à la seconde extrémité du chariot, chaque fois que l'une desdites première ou seconde extrémités de chariot forme au moins une partie de ladite paroi du récipient.

12. Chariot (1) selon l'une quelconque des revendications précédentes, dans lequel chaque conteneur de stockage comprend une partie de support destinée à supporter un conteneur de stockage ou une partie de la surface de travail ci-dessus.

13. Chariot (1) selon l'une quelconque des revendications précédentes, dans lequel chaque partie de support comprenant une partie de guidage, comprenant en particulier au moins une roue, pour guider un récipient de stockage ou une surface de travail ci-dessus, plus particulièrement dans lequel les récipients de stockage comprennent partie de guidage complémentaire, notamment un rail de guidage pour au moins une roue.

14. Chariot (1) selon l'une quelconque des revendications précédentes, dans lequel extrémités du récipient de stockage opposées à l'extrémité du récipient de stockage reliées à une extrémité du chariot sont reliées aux extrémités du récipient de stockage correspondantes des extrémités du récipient de stockage supérieure ou inférieure afin de fournir une butée au niveau de ladite position étendue.

15. Chariot (1) selon l'une quelconque des revendications précédentes, dans lequel ledit chariot est sensiblement rectangulaire, lesdites extrémités du chariot définissant des plans d'extrémité opposés dudit chariot, et lesdites extrémités du chariot étant couplées de manière coulissante l'une à l'autre pour coulisser dans une direction sensiblement normale dudit plan d'extrémité.
